# FASCICULE DE BREVET EUROPEEN

(11) **EP 2 349 227 B1**
(45) Date de publication et mention de la délivrance du brevet: **30.05.2018**
(21) Numéro de dépôt: 09783697.7
(22) Date de dépôt: 02.10.2009
(51) Int. Cl.: A61K 9/50

(54) **FORME PHARMACEUTIQUE ORALE A BASE DE MICROGRANULES A LIBERATION PROLONGEE RESISTANTE A L'ALCOOL**
Alkohol-resistentes Mikrogranulat
ORAL PHARMACEUTICAL FORM BASED ON MICROGRANULATES WITH PROLONGED RELEASE RESISTANT TO ALCOHOL

(30) Priorité: 02.10.2008 FR 0856660
(43) Date de publication de la demande: 03.08.2011
(73) Titulaire: ETHYPHARM, 92210 Saint-Cloud (FR)
(72) Inventeur: HERRY, Catherine, F-76320 Saint-Pierre-lès-Elbeuf (FR); TRICHARD, Laury, F-78000 Versailles (FR)
(74) Mandataire: Regimbeau
(86) Numéro de dépôt international: PCT/EP2009/062836
(87) Numéro de publication internationale: WO 2010/037854

(56) Documents cités:
- WO-A-02/065834
- WO-A-02/096392
- WO-A-2004/010983
- WO-A-2006/047493
- WO-A-2007/109357
- WO-A2-2007/093642
- US-A1- 2006 105 039
- US-A1- 2007 065 512
- US-A1- 2007 092 568
- US-A1- 2007 196 491
- US-A1- 2008 003 286
- US-A1- 2008 233 197

## Description

L'invention a pour objet l'utilisation d'une forme pharmaceutique orale à base de microgranules à libération prolongée d'au moins un principe actif, cette forme étant résistante à la décharge immédiate de la dose de principe actif en présence d'alcool.

De nombreuses formes pharmaceutiques à libération prolongée pour administration orale existent sur le marché. La libération du principe actif doit être contrôlée en fonction de l'objectif thérapeutique et des propriétés pharmacologiques du principe actif. Certains principes actifs peuvent se révéler très toxiques, voire mortels, si la dose ingérée dépasse un certain seuil.

Il est donc impératif que leurs propriétés « retard » soient étroitement contrôlées, et ceci afin de s'assurer qu'une libération rapide du principe actif, ou « dose-dumping », ne puisse pas se produire, notamment lors d'une prise concomitante d'alcool. La consommation d'une grande quantité d'alcool en même temps qu'une prise de médicament peut en effet altérer la forme pharmaceutique qui libère alors très rapidement la totalité du principe actif qu'elle contient.

La demande PCT WO9611675 décrit des microcapsules à libération modifiée pour l'administration per os de principes actifs médicamenteux et/ou nutritionnels (PA), dont la taille est inférieure ou égale à 1000 µm. Ces microcapsules sont constituées par des particules enrobées par un matériau d'enrobage constitué par un mélange d'un polymère filmogène (éthylcellulose), d'un agent plastifiant hydrophobe (huile de ricin), d'un agent tensioactif ou lubrifiant (stéarate de magnésium) et d'un polymère azoté (polyvinylpyrrolidone : povidone, PVP). Ces microcapsules sont également caractérisées par leur aptitude à séjourner longtemps (au moins 5 h) dans l'intestin grêle et à permettre, lors de ce séjour, l'absorption du PA sur une période supérieure au temps de transit naturel dans l'intestin grêle.

La demande PCT WO2007093642 décrit une forme pharmaceutique orale multiparticulaire d'un diamètre moyen inférieur à 2000 µm, constituée au moins d'un polymère hydrophile, d'un coeur comprenant le principe actif et enrobé par un enrobage comprenant au moins un polymère insoluble dans les liquides du tube digestif, au moins un agent plastifiant et éventuellement au moins un agent tensioactif.

Afin d'évaluer la résistance à l'alcool des compositions pharmaceutiques, la FDA (Food And Drug Administration) suggère la réalisation de tests de dissolution *in vitro* pour comparer les cinétiques obtenues dans le milieu HCl 0,1N (représentatif du pH gastrique) avec les cinétiques obtenues dans le même milieu substitué à 5, 20 et 40% (v/v) par de l'éthanol. D'après Walden et al. (The Effect of Ethanol on the Release of Opioids from Oral Prolonged-Release Preparations Drug Development and Industrial Pharmacy, 33:10, 1101 - 1111, 2007), le fait d'exposer *in vitro* une forme pharmaceutique sur une période de 2h est considérée comme représentative de la durée d'exposition de ces formes pharmaceutiques *in vivo.*

Un objectif essentiel de la présente invention est de proposer une forme pharmaceutique à base de microgranules à libération prolongée d'au moins un principe actif destinée à l'administration par voie orale, permettant d'éviter ou de limiter une décharge immédiate du principe actif induite par la consommation d'alcool lors de l'administration de cette forme pharmaceutique.

Définitions au sens du présent exposé de l'invention :

### Support neutre

On entend par "support neutre" ou "noyau neutre" ou encore plus simplement "neutre", des supports inertes sphériques ou quasi-sphériques de taille comprise entre 50 µm et 3 mm et préférentiellement entre 100 et 1000 µm tels que ceux habituellement utilisés dans l'industrie pharmaceutique comme support de base de principes actifs pour la constitution de microgranules par exemple.

### Support neutre rendu insoluble

On entend par support neutre rendu insoluble dans l'eau ou dans une solution alcoolique, un support neutre constitué par des matériaux solubles dans l'eau ou dans une solution alcoolique recouvert d'au moins une couche de matériaux insolubles dans l'eau ou dans une solution alcoolique et dont la fonction est de limiter, voire d'empêcher la pénétration de ces dits milieux vers le coeur du support.

### Microgranules

Les microgranules de la présente invention se rapportent à des unités galéniques sphériques, constitués en leur centre d'un support neutre, recouvert d'au moins une couche contenant le principe actif qui est elle-même recouverte d'au moins une couche polymérique.

### Libération prolongée

Dans la présente demande, on utilisera le terme libération prolongée pour désigner un profil de libération du principe actif modifié par rapport à celui qu'aurait présenté le principe actif seul dans un système à libération immédiate comme défini par la Pharmacopée Européenne (quantité de principe actif libérée en 45 minutes au moins égale à 75%, Ph. Eur., 6^{ème} édition 2.9.3.)

### Alcool

Le terme " alcool " représente l'éthanol et les termes " solution alcoolique " ou " milieu alcoolique " représente une solution aqueuse d'éthanol.

Le but de la présente invention est d'offrir une nouvelle composition pharmaceutique orale à base de microgranules constituées de trois parties distinctes. Ces microgranules sont ainsi constitués, du centre vers la périphérie par au moins un support neutre rendu insoluble dans l'eau ou dans une solution alcoolique puis par au moins une couche active comprenant le ou les principes actifs et enfin au moins d'une couche comprenant au moins un polymère d'intérêt, c'est à dire un polymère dont on veut exploiter les propriétés pour influer sur le profil de libération du principe actif.

Dans l'art antérieur, par exemple dans le document WO2007093642 les microparticules qui permettent la libération prolongée du principe actif dans une solution alcoolique sont généralement constitués d'un coeur de principe actif pouvant être du principe actif brut (pur) sous forme pulvérulente, et/ou un granulé matriciel de principe actif mélangé à différents autres ingrédients, et/ou un support neutre, par exemple en cellulose ou en sucre, recouvert d'au moins une couche comportant du principe actif. Cependant ce document prévoit l'incorporation systématique d'un composé pharmaceutiquement acceptable dont la vitesse ou la capacité à s'hydrater ou à se solvater est supérieure en milieu aqueux exempt d'alcool qu'en solution alcoolique (l'agent D), de différentes façons (dans le coeur de principe actif, dans l'enrobage des microparticules et/ou la phase liante de granules). Or l'exemple 3 de la présente invention démontre que l'incorporation d'un agent D dans la formulation de microgranules selon les proportions revendiquées dans le document WO2007093642 peut entraîner la perte de l'effet de libération prolongée dans un milieu dépourvu d'alcool. L'incorporation d'un agent D peut donc être un inconvénient majeur pour formuler des microgranules LP résistants à l'alcool.

De plus, le document WO2007093642 n'enseigne pas que l'utilisation d'un neutre insoluble dans l'eau en présence d'un enrobage polymérique constitué uniquement d'un polymère de nature hydrophobe est adaptée à la libération prolongée du principe actif dans une solution alcoolique.

C'est pourquoi, le problème technique que se propose de résoudre l'invention tient dans l'utilisation d'une forme pharmaceutique à base de microgranules à libération prolongée résistante à l'alcool comprenant un neutre rendu insoluble dans l'eau ou dans une solution alcoolique en présence d'un enrobage polymérique à effet retard.

### BREVE DESCRIPTION DES FIGURES

Figure 1 : Profils de dissolution du Diltiazem HCl à partir des neutres de sucre enrobés d'éthylcellulose + TEC (CL 10%) dans différents milieux.
Figure 2 : Profils de dissolution du Diltiazem HCl à partir des neutres de cellulose enrobés d'éthylcellulose + TEC (CL 10%) dans différents milieux.
Figure 3 : Profils de dissolution du Diltiazem HCl à partir des neutres de cellulose enrobés d'éthylcellulose + TEC (CL 20%) dans différents milieux.
Figure 4 : Profils de dissolution du Diltiazem HCl à partir des microgranules préparées selon l'invention WO2007093642 dans différents milieux (neutres de cellulose).
Figure 5 : Profils de dissolution du Diltiazem HCl à partir des neutres de sucre rendus insolubles et enrobés d'éthylcellulose + TEC (CL 20%) dans différents milieux.
Figure 6 : Profils de dissolution du Diltiazem HCl à partir des neutres de cellulose enrobés d'éthylcellulose + Triacétine (CL 20%) dans différents milieux.
Figure 7 : Profils de dissolution du Diltiazem HCl à partir des neutres de cellulose enrobés d'éthylcellulose + Myvacet 9-45 (CL 20%) dans différents milieux.
Figure 8 : Profils de dissolution du Diltiazem HCl à partir des neutres de cellulose enrobés d'éthylcellulose + DBS (CL 20%) dans différents milieux.
Figure 9 : Profils de dissolution du Carvedilol Phosphate à partir des neutres de sucre enrobés d'éthylcellulose + TEC (CL 5%) dans différents milieux.
Figure 10 : Profils de dissolution du Carvedilol Phosphate à partir des neutres de cellulose enrobés d'éthylcellulose + TEC (CL 5%) dans différents milieux.
Figure 11 : Profils de dissolution du Carvedilol Phosphate à partir des neutres de sucre rendus insoluble et enrobés d'éthylcellulose + TEC (CL 5%) dans différents milieux.
Figure 12 : Profils de dissolution de granules de Tolterodine enrobés avec Surelease + hypromellose dans différents milieux

### DESCRIPTION DETAILLEE DE L'INVENTION

La forme pharmaceutique orale selon l'invention, comprend des microgranules à libération prolongée d'au moins un principe actif, chaque microgranule comprenant un support neutre rendu insoluble dans l'eau ou dans une solution alcoolique, comportant au moins une première couche de montage comprenant au moins un principe actif et éventuellement un agent liant pharmaceutiquement acceptable, l'ensemble comportant au moins un enrobage à base d'au moins un polymère hydrophobe et un plastifiant. Le polymère hydrophobe empêche la libération immédiate du principe actif. Le support neutre rendu insoluble est obtenu en recouvrant un support neutre d'un ou plusieurs excipients de nature hydrophobe et choisis parmi les dérivés de la cellulose.

Optionnellement, la forme pharmaceutique de l'invention comprend au moins un agent tensio-actif de préférence dans la couche de montage. La forme pharmaceutique selon l'invention est pour utilisation dans une méthode thérapeutique pour éviter ou limiter une décharge immédiate du principe actif induite par la consommation d'alcool. La forme pharmaceutique orale selon l'invention est préférentiellement résistante à la décharge immédiate de la dose de principe actif à l'alcool, et est caractérisée en ce que le pourcentage d'actif libéré après 2h dans un milieu acido-alcoolique HCl 0,1N contenant de l'alcool et de préférence une quantité d'éthanol comprise entre 4 et 30%, n'est pas supérieur de plus de 15 points (15% en valeur absolue) comparé à celui libéré dans un milieu de acide HCl 0,1N.

De préférence, le ou les principes actifs sont intégrés dans la couche active en association avec un agent liant pharmaceutiquement acceptable, tel que ceux habituellement utilisés dans l'industrie pharmaceutique pour la fixation de principes actifs à la surface de supports neutres. Ainsi, la méthode de fixation de la couche active décrite dans le brevet EP 1 200 071 peut tout à fait être employée pour la fixation de la couche active dans le cadre de la présente invention.

De façon préférée, la couche active des microgranules conformes à l'invention est appliquée par pulvérisation d'une dispersion de principe actif dans un solvant (appelée dispersion de montage). Avantageusement, cette dispersion contient également l'agent liant.

Parmi les agents liants pharmaceutiquement acceptables, on utilisera préférentiellement des agents liants de nature hydrophile et notamment des dérivés de la cellulose tels que l'HPMC, en particulier les grades Pharmacoat® 603 et Pharmacoat® 606, des dérivés de la polyvinylpyrrolidone, en particulier le grade PVP K 30 et également des dérivés du polyéthylene glycol, en particulier le polyéthylene glycol dont le poids moléculaire vaut entre 3000 et 7000, tels que le PEG4000 et le PEG6000 notamment, et leurs mélanges.

Le solvant de la dispersion de montage pulvérisée doit être adapté au principe actif ou au mélange de principes actifs employés. Ainsi, on pourra par exemple utiliser l'eau, des solvants organiques, parmi eux l'éthanol ou des solutions hydro-alcooliques de diverses concentrations pour la réalisation de la solution à la base de la couche active.

Un agent tensioactif peut être ajouté à la phase de montage pour améliorer la solubilité du principe actif ou stabiliser la suspension de montage. L'agent tensioactif est utilisé en quantités de 0 à 50% et préférentiellement entre 0 et 20%. Parmi les tensioactifs utilisables, peuvent être cités les sels alcalins ou alcalinoterreux des acides gras, le sodium dodécyl sulfate et le docusate de sodium étant préférés, les huiles polyoxyéthylénées, de préférence l'huile de ricin hydrogénée polyoxyéthylénée, les copolymères polyoxyéthylène-polyoxypropylène, les esters de sorbitan polyoxyéthylénés, les dérivés de l'huile de ricin polyoxyéthylénés, les stéarates, de préférence de calcium, de magnésium, d'aluminium ou de zinc, les polysorbates, les stéarylfumarates, de préférence de sodium, le béhénate de glycérol, le chlorure de benzalkonium, le bromure d'acétyltriméthyl ammonium, l'alcool cétylique et leurs mélanges.

Dans la mesure du possible, il est préférable d'utiliser des solvants non toxiques et facilement éliminables par évaporation lors du séchage afin qu'il n'en subsiste aucune trace dans les microgranules.

L'enrobage permettant de contrôler la libération contient un polymère hydrophobe empêchant la libération immédiate du principe actif en quantité comprise entre 50% à 100%, de préférence de 70% à 100%, de la masse sèche de ladite couche d'enrobage.

Le taux d'enrobage représente le rapport de la quantité de masse sèche constituant l'enrobage assurant une libération prolongée du principe actif sur la masse totale du microgranule avant enrobage (en masse sèche). Le taux d'enrobage est compris entre 0,1% à 50% m/m, de préférence, de 2% à 30% m/m, et, plus préférentiellement encore, de 5% à 30%. Ou autrement dit, le rapport entre la masse de vernis sec (= polymère et éventuels additifs en masse sèche) constituant l'enrobage empêchant la libération immédiate du principe actif sur la masse totale du microgranule avant enrobage (en masse sèche) est compris entre 0,1% à 50% m/m, de préférence, de 2% à 30% m/m, et, plus préférentiellement encore, de 5% à 30%.

Les polymères utilisés pour assurer une libération prolongée du principe actif sont des polymères de nature hydrophobe, de préférence, sélectionnés dans le groupe de produits suivants : les dérivés non hydrosolubles de la cellulose, les dérivés de (co)polymères (méth)acryliques, les dérivés des polyvinylacétates et leurs mélanges.

Plus préférentiellement, le ou les polymère(s) hydrophobe(s) empêchant la libération immédiate du principe actif est (sont) sélectionné(s) dans le groupe de produits suivants : l'éthylcellulose, l'acétate butyrate de cellulose, l'acétate de cellulose, les copolymères ammonio-méthacrylates type A et type B vendus sous le nom commercial Eudragit®, notamment l'Eudragit® RS 30D, l'Eudragit NE 30D, l'Eudragit® RL 30D, l'Eudragit® RS PO et l'Eudragit® RL PO de la famille des poly(éthyl acrylate, méthyl méthacrylate, triméthylamonioéthyl méthacrylate), les polyvinylacétates et leurs mélanges.

Lorsque l'enrobage est réalisé en voie aqueuse, un agent plastifiant peut être ajouté à la dispersion d'enrobage à raison de 0% à 50% m/m, de préférence, de 2% à 25% m/m, en masse sèche de polymère d'enrobage.

L'agent plastifiant est sélectionné notamment dans le groupe de produits suivants : le glycérol et ses esters, de préférence dans le sous-groupe suivant : les triglycérides à chaînes moyennes, les glycérides acétylés, glycéryl-monostéarate, glycéryl-triacétate, glycéryl-tributyrate, les phtalates, de préférence dans le sous-groupe suivant : dibutylphtalate, diéthylphtalate, diméthylphtalate, dioctylphtalate, les citrates, de préférence dans le sous-groupe suivant : acétyltributylcitrate, acétyltriéthylcitrate, tributylcitrate, triéthylcitrate, les sébaçates, de préférence dans le sous-groupe suivant : diéthylsébaçate, dibutylsébaçate, les adipates, les azélates, les benzoates, le chlorobutanol,les polyéthylène glycols, les huiles végétales, les fumarates, de préférence le diéthylfumarate, les malates, de préférence le diéthylmalate, les oxalates, de préférence le diéthyloxalate, les succinates ; de préférence le dibutylsuccinate, les butyrates, les esters de l'alcool cétylique, les malonates, de préférence le diéthylmalonate, l'huile de ricin (celle-ci étant particulièrement préférée), et leurs mélanges.

Plus préférentiellement, l'agent plastifiant est sélectionné dans le groupe de produits suivants : les monoglycérides acétylés notamment le Myvacet® 9-45, le triéthylcitrate (TEC), le dibutylsébacate, la triacétine et leurs mélanges.

L'agent tensioactif est optionnellement présent dans l'enrobage à raison de 0 à 30% m/m, de préférence de 0 à 20% m/m, et, plus préférentiellement encore, de 5 à 15% de la masse sèche de plastifiant. L'agent tensioactif est de préférence sélectionné dans le groupe de produits suivants : les sels alcalins ou alcalinoterreux des acides gras, le sodium dodécyl sulfate et le docusate de sodium étant préférés, les huiles polyoxyéthylénées, de préférence l'huile de ricin hydrogénée polyoxyéthylénée, les copolymères polyoxyéthylène-polyoxypropylène, les esters de sorbitan polyoxyéthylénés, les dérivés de l'huile de ricin polyoxyéthylénés, les stéarates, de préférence de calcium, de magnésium, d'aluminium ou de zinc, les polysorbates, les stéarylfumarates, de préférence de sodium, le béhénate de glycérol, le chlorure de benzalkonium, le bromure d'acétyltriméthyl ammonium, l'alcool cétylique et leurs mélanges.

Une charge inerte peut être présente dans l'enrobage à raison de 0 à 50% m/m, de préférence de 0 à 20% m/m, et, plus préférentiellement encore, de 5 à 20% de la masse sèche du polymère enrobage.

La charge inerte uniformément répartie dans l'enrobage est choisie dans le groupe comprenant notamment le talc, la silice colloïdale anhydre, le stéarate de magnésium, le monostéarate de glycérol et leurs mélanges.

Le phénomène de résistance à la libération immédiate du principe actif observé par la demanderesse dans un milieu acido-alcoolique montre une dépendance suivant la nature du neutre utilisé et le taux d'enrobage des microgranules.

### Le principe actif

La couche active constituant les microgranules conformes à l'invention comprend au moins un principe actif pharmaceutique qui peut être de toute nature.

Les microgranules selon la présente invention peuvent comprendre en tant que principe actif, les hormones ou leurs dérivés par exemple, les principes actifs agissant sur le système nerveux central, les principes actifs agissant sur le système cardiovasculaire, les antibiotiques, les anti-viraux et les analgésiques.

Les principes actifs agissant sur le système nerveux central sont de préférence choisis parmi les anti-épileptiques, les anti-parkinsonniens, les psycho-stimulants, les psychotropes, les antidépresseurs, les anxiolytiques et les anti-psychotiques par exemple.

Les principes actifs agissant sur le système cardiovasculaire sont de préférence choisis parmi les anti-hypertenseurs, les anti-thrombotiques, les anti-agrégants et les hypocholestérolémiants notamment.

Les antibiotiques peuvent être choisis parmi les bêta-lactamines, les cyclines, les aminosides, les macrolides, les quinolones, les antibiotiques glycopeptidiques, le imidazolées, les sulfamides, les antituberculeux et les antilépreux notamment.

Les antiviraux peuvent être choisis parmi les inhibiteurs de la réplication ou de la multiplication virale notamment.

Les analgésiques peuvent être choisis parmi les analgésiques non opiacés, opiacés faibles, opioïdes mixtes, morphiniques ou spasmodiques, notamment l'hydrocodone, l'hydromorphone, la morphine, l'oxycodone, l'oxymorphone, le tramadol, la gabapentine et leurs dérivés.

### Le procédé de préparation des microgranules

La présente invention a en outre pour objet le procédé de préparation des microgranules précédemment décrits qui comprend les étapes suivantes :- l'introduction de supports sphériques neutres insolubles ou rendus insolubles dans une enceinte réactionnelle à lit fluidisé, - la pulvérisation sur ces supports sphériques neutres d'au moins un principe actif en solution ou en suspension dans un solvant organique et/ou aqueux additionné d'au moins un polymère hydrosoluble ou non hydrosoluble (agent liant), - la pulvérisation d'une suspension d'enrobage comprenant au moins un polymère hydrophobe sur les particules enrobées obtenues à l'étape précédente,- éventuellement, le séchage des microgranules médicamenteux ainsi obtenus.

### Préparation de la dispersion de montage

L'étape dite de montage de la couche active conformément à la présente invention permet d'obtenir des microgranules dont la teneur en actif est à la fois précise et uniforme.

La dispersion dite de montage est la dispersion dans laquelle le ou les principes actifs vont être dissous ou mis en suspension (dispersés) et qui va être pulvérisée à la surface des microgranules. Cette dispersion contient avantageusement un agent liant conventionnel dissous également.

### Montage de la couche active

Le principe actif est appliqué sur les granules de façon conventionnelle par pulvérisation, en lit fluidisé ou en turbine perforée par exemple. D'une manière générale, ce procédé repose sur la pulvérisation simultanée au travers d'une buse, du ou des principes actifs et éventuellement d'un liant qui sont dissous ou dispersés dans la solution de montage, ce qui garantit pour cette étape du procédé une parfaite homogénéité de teneur.

Le temps nécessaire au montage est très variable et dépend de la quantité d'actif à pulvériser et de sa solubilité dans la solution de montage. De manière générale il est compris entre 1 et 10 heures.

A l'issue de l'étape de montage, les microgranules sont séchés en lit fluidisé ou en turbine perforée puis tamisés.

### Enrobage des microgranules

Le polymère d'enrobage est appliqué sur les microgranules précédents de façon conventionnelle par pulvérisation, en lit fluidisé ou en turbine perforée par exemple. D'une manière générale, ce procédé repose sur la pulvérisation simultanée au travers d'une buse, du ou des polymères d'enrobage et éventuellement d'un plastifiant et/ou d'un agent tensioactif et/ou d'une charge inerte qui sont dissous ou dispersés dans un solvant adapté.

Une solution organique de polymère peut être utilisée pour l'enrobage : dans ce cas, le procédé consiste en la pulvérisation de la solution et un séchage dans le même équipement.

Si le véhicule est l'eau, une dispersion aqueuse de polymère est utilisée, un plastifiant doit être ajouté pour améliorer la qualité de l'enrobage. Le procédé consiste alors en la pulvérisation de la dispersion, un séchage dans le même équipement et, si nécessaire, une étape de maturation du film d'enrobage (aussi appelée curing) qui permet l'obtention d'un film homogène et uniforme. Le curing peut être réalisé en lit fluidisé, en turbine perforée ou en étuve par exemple.

Le temps nécessaire à l'enrobage est très variable et dépend de la quantité de polymère à pulvériser. De manière générale il est compris entre 1 et 10 heures.

A l'issue de l'étape d'enrobage, les microgranules sont séchés en lit d'air fluidisé puis tamisés.

### Les tests de dissolution et de dosage

D'une manière générale, les conditions de dosage et de dissolution des microgranules conformes à l'invention sont celles prescrites par les différentes pharmacopées, notamment européenne, américaine ou japonaise.

Ainsi, pour déterminer les cinétiques de libération des différents systèmes étudiés, un appareil de dissolution thermostaté conventionnel à palettes ou à paniers peut être employé. Les unités médicamenteuses sont introduites dans chaque vase et des prélèvements sont effectués périodiquement pour déterminer la quantité de principe actif libéré au cours du temps. Les prélèvements peuvent être manuels ou automatiques et les analyses peuvent être réalisées directement avec un spectrophotomètre UV/visible ou après séparation en CLHP (chromatographie liquide haute performance) couplée à une détection UV/visible par exemple.

Selon un autre aspect, l'invention porte sur l'utilisation d'une forme pharmaceutique orale à base de microgranules à libération prolongée, comprenant un support neutre insoluble dans l'eau ou dans une solution alcoolique, ou un neutre rendu insoluble dans l'eau ou dans une solution alcoolique, comportant au moins un montage comprenant au moins un principe actif et éventuellement un agent liant ; l'ensemble comportant au moins un deuxième enrobage à base d'au moins un polymère hydrophobe empêchant la libération immédiate du principe actif, ladite forme pharmaceutique étant destinée à éviter ou limiter une décharge immédiate du principe actif induite par la consommation d'alcool lors de l'administration de cette forme pharmaceutique.

### EXEMPLES

### Exemple 1 : Microgranules de Diltiazem HCl à libération prolongée résistants à l'alcool (taux d'enrobage de 10%) (exemple de référence)

### a) Préparation des microgranules de Diltiazem HCl à libération prolongée

Le principe actif utilisé est le chlorhydrate de diltiazem (Zambon), de formule C₂₂H₂₆N₂O₄S, HCl.

Les noyaux neutres utilisés sont des sphères de sucre (Suglets® 30 NPPHARM) et des sphères de cellulose (Ethisphères® 600 NPPHARM). La taille de ces supports est de l'ordre de 500 à 700 µm.

L'agent liant utilisé est la polyvinylpyrrolidone (PVP K 30, BASF). Elle est solubilisée dans l'eau puis le Diltiazem HCl est ajouté à cette solution aqueuse, constituant la solution de montage.

La composition de solution de montage et les quantités de matières mises en oeuvre pour l'étape de montage sont les suivantes :

| | Quantité (grammes) | | Composition centésimale en matière sèche (%) |
|---|---|---|---|
| | Neutres de sucre | Neutres de cellulose | |
| Noyaux neutres | 800 | 1500 | 78 |
| PVP K 30 | 25 | 47 | 2 |
| Diltiazem HCl | 200 | 375 | 20 |
| Eau purifiée | 800 | 1500 | N/A |
| Total en masse sèche | 1025 | 1922 | 100 |

| | | | |
|---|---|---|---|
| N/A : non applicable | | | |

Les noyaux sont introduits dans un lit fluidisé (OHLMANN) équipé d'un Würster. La solution de montage est pulvérisée en bottom spray avec une buse de diamètre 1,2 mm. Un tamisage avec des grilles d'ouverture de mailles 500 et 900 µm est réalisé après le montage afin d'éliminer respectivement les fines et les doubles.

La suspension d'enrobage est réalisée à partir d'une préparation commerciale d'Aquacoat® ECD 30 (FMC) qui est une dispersion aqueuse contenant 29 à 32% de matière sèche dont 24,5 à 29,5% d'ethylcellulose, de 0,9 à 1,7% de lauryl sulfate de sodium et de 1,7 à 3,3% d'alcool cétylique. A cette suspension est ajouté un plastifiant, le triéthylcitrate (Vertellus). La suspension est ensuite diluée afin d'obtenir une dispersion aqueuse à 15% de masse sèche.

La composition de la suspension d'enrobage finale est la suivante :

| | Composition centésimale(%) | Composition centésimale en matière sèche (%) |
|---|---|---|
| Aquacoat® ECD 30 | 40,3 | 15 |
| *(masse sèche)* | *(12,09)* | |
| Triéthylcitrate | 2,9 | |
| Eau purifiée | 56,8 | N/A |

La suspension d'enrobage est pulvérisée sur les microgranules montés avec le Diltiazem HCl.

Les quantités de matières mises en oeuvre pour l'étape d'enrobage sont les suivantes :

| | Quantité (grammes) pour obtenir un taux d'enrobage de 10% |
|---|---|
| Noyaux montés avec le Diltiazem HCl | 900 |
| Suspension d'enrobage à 15% matière sèche | 600 |
| *(masse sèche)* | 90 |
| Total en masse sèche | 990 |

L'enrobage est réalisé en lit fluidisé (OHLMANN), équipé d'un Würster. La pulvérisation s'effectue en bottom spray avec une buse de diamètre 1,2 mm. Un tamisage avec des grilles d'ouverture de mailles 500 µm et 1000 µm est réalisé après l'enrobage afin d'éliminer respectivement les fines et les doubles.

Une maturation du film d'enrobage est réalisée en étuve (FIRLABO) pendant 24h à 60°C et avec 75% d'humidité relative (HR).

Les compositions qualitative et quantitative des microgranules de Diltiazem HCl sont récapitulées dans le tableau suivant.

| | | Composition centésimale pour obtenir un taux d'enrobage de 10% (%) |
|---|---|---|
| | Support neutre | 70,6 |
| **Montage** | PVP K30 | 2,2 |
| | Diltiazem HCl | 17,6 |
| **Enrobage** | Aquacoat ECD 30 *(en masse sèche)* | 7,3 |
| | Triéthylcitrate | 1,8 |
| **Lubrification** | Aérosil® R972 | 0,5 |

### b) Dosage et dissolution des microgranules

Les essais de libération *in vitro* du principe actif sont réalisés dans un appareil de dissolution à pales tournantes (Pharmacopée Européenne, Sotax AT7, logiciel IDIS). L'analyse se fait avec un spectrophotomètre UV / visible à une longueur d'onde de 237 nm (Kontron Instruments spectrophotometer, UVIKON 922).

Les échantillons sont soumis à une agitation constante dans des vases contenant chacun 900mL de milieu de dissolution, et la température est maintenue constante à 37°C (+/-0,5°C), Les milieux de dissolution utilisés sont composés soit d'HCl 0,1N, soit d'un mélange HCl 0,1N / Ethanol absolu avec une concentration en éthanol absolu égale à 10 ou 20% (v/v). La vitesse de rotation des pales est fixée à 100 tours/min.

Des prélèvements sont réalisés en continu sur 24h dans chacun des 6 vases de l'appareil. Pour chaque lot, le test est réalisé sur 3 vases, avec 3 prises d'essai de microgranules équivalentes chacune à 150mg de PA.

### c) Profils obtenus en fonction des deux types de supports

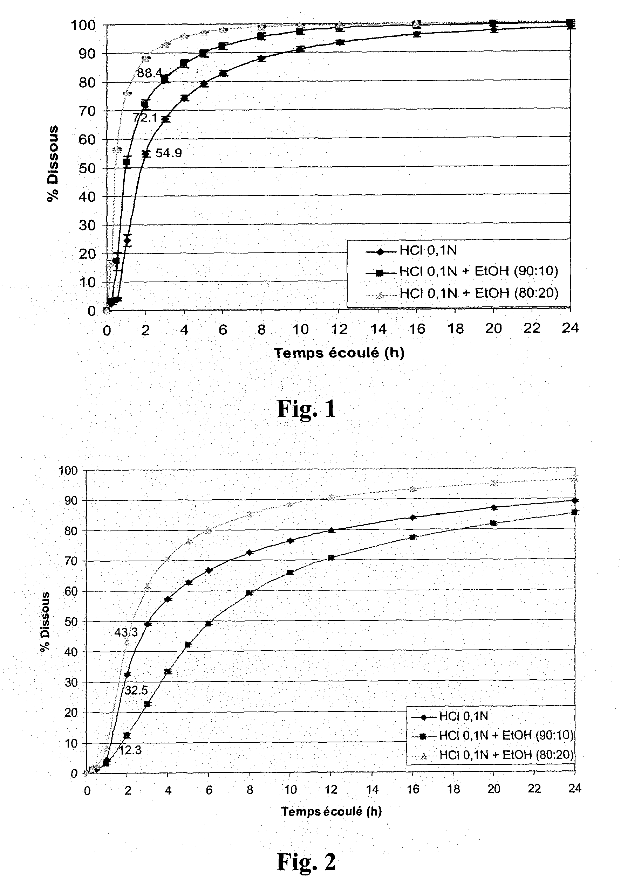

Les profils de dissolution obtenus dans l'HCl 0,1N et les mélanges HCl 0,1N / Ethanol absolu concentrés à 10 et 20% (v/v) en éthanol absolu pour les microgranules présentant un taux d'enrobage de 10% sont consignés sur les figures 1 et 2.

La figure 1 montre que la vitesse de libération du Diltiazem HCl monté sur supports de sucre est accélérée avec l'augmentation de la concentration en éthanol par rapport au milieu composé d'HCl 0,1N seul. Le profil obtenu en présence de concentration d'alcool à 20 (v/v) est un profil immédiat. Il y a donc perte de l'effet prolongé en présence d'alcool pour cette formulation de microgranules LP. A l'inverse, les systèmes à base de neutres de cellulose présentent en effet une libération prolongée dans les milieux ayant une concentration en éthanol de 0, 10 et 20% (v/v).

L'écart des pourcentages de Diltiazem HCl libéré dans les milieux acido-alcooliques ou dans l'HCl 0,1N pour chaque système étudié peut être calculé. Le tableau suivant présente ces écarts pour le temps 2h, qui est le plus représentatif d'une résistance à l'alcool en se basant sur la vitesse d'absorption gastro-intestinale de l'éthanol (Cmax ≤ 2h), d'après l'expertise collective INSERM (2001).

| | | **Ethanol 10% (%)** | **Ethanol 20% (%)** |
|---|---|---|---|
| Neutres de sucre | écart à t(2h) | 17 | 34 |
| Neutres de cellulose | écart à t(2h) | -20 | 11 |

Pour les systèmes à base de neutres de sucre, tous les écarts sont supérieurs à 15%. En revanche, les écarts observés avec les systèmes à base de neutres de cellulose sont inférieurs à 15% pour les deux concentrations en éthanol. Le système utilisant la cellulose pour support semble donc présenter un potentiel plus important pour le développement de formes à libération prolongée résistantes à l'alcool que le système utilisant le sucre.

### Exemple 2 : Microgranules de Diltiazem HCl à libération prolongée résistants à l'alcool (taux d'enrobage de 20%) (exemple de référence)

Selon une variante de l'exemple 1, les microgranules de Diltiazem HCl résistants à l'alcool peuvent être obtenus en réalisant un taux d'enrobage de 20%.

Les méthodes de préparation, de dosage et de dissolution des microgranules restent identiques à l'exemple 1, à l'exception de leur composition quantitative récapitulée dans le tableau suivant.

| | | Composition centésimale pour obtenir un taux d'enrobage de 20% |
|---|---|---|
| **Montage** | Support neutre de cellulose | 64,7 |
| | PVP K30 | 2,0 |
| | Diltiazem HCl | 16,2 |
| **Enrobage** | Aquacoat ECD 30 *(en masse sèche)* | 13,4 |
| | Triéthylcitrate | 3,2 |
| **Lubrificatio n** | Aérosil® R972 | 0,5 |

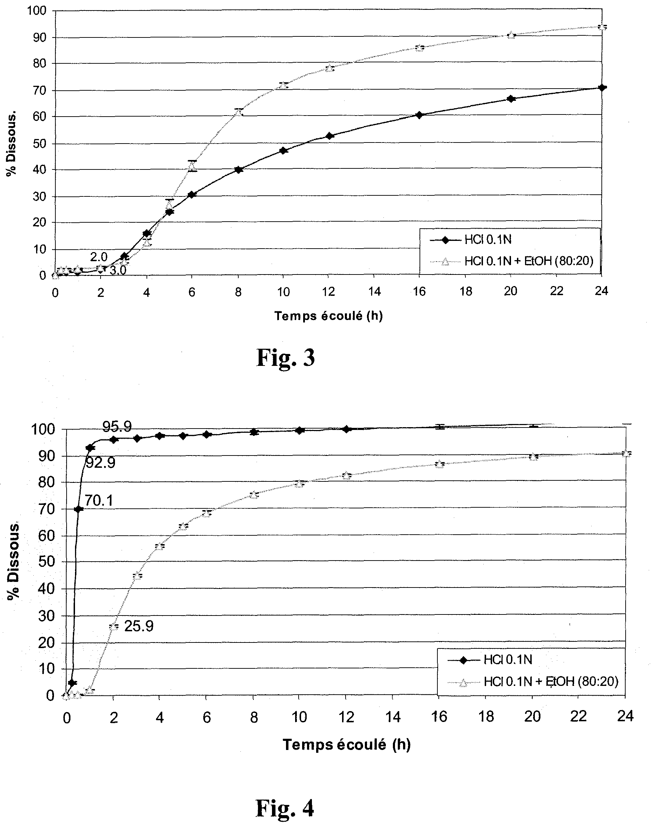

Les profils de dissolution obtenus dans l'HCl 0,1N et le mélange HCl 0,1N / Ethanol absolu concentré à 20 % (v/v) en éthanol absolu pour les microgranules présentant un taux d'enrobage de 20% sont consignés sur la figure 3.

L'écart des pourcentages d'actif libéré au bout de 2 heures dans l'HCl 0,1N par rapport au milieu acido-alcoolique est de 1%, ce qui démontre que ces microgranules LP sont résistants à l'alcool.

### Exemple 3 : Microgranules de Diltiazem HCl incorporant un agent D selon le brevet WO2007093642, (exemple de référence)

Selon une variante de l'exemple 2, un composé pharmaceutiquement acceptable dont la vitesse ou la capacité à s'hydrater ou à se solvater est supérieure en milieu aqueux exempt d'alcool qu'en solution alcoolique, décrit comme « agent D » dans le document WO2007093642, a été incorporé à la formulation des microgranules. Selon le deuxième mode de réalisation de l'invention WO2007093642, l'agent D a été incorporé dans l'enrobage, à raison de 10% de la masse totale de cet enrobage, comme décrit préférentiellement dans ce document.

Les méthodes de préparation, de dosage et de dissolution des microgranules restent identiques à l'exemple 1. L'agent D est incorporé à la suspension d'enrobage contenant le polymère d'enrobage et le plastifiant. La composition quantitative des microgranules est récapitulée dans le tableau suivant :

| | | Composition centésimale pour obtenir un taux d'enrobage de 20% |
|---|---|---|
| | Support neutre de cellulose | 60,7 |
| **Montage** | PVP K30 | 1,9 |
| | Diltiazem HCl | 15,2 |
| **Enrobage** | Aquacoat ECD 30 *(en masse sèche)* | 15,5 |
| | Triéthylcitrate | 3,9 |
| | HPMC Pharmacoat 603 (agent D) | 2,3 |
| **Lubrificatio n** | Aérosil® R972 | 0,5 |

Les profils de dissolution obtenus dans l'HCl 0,1N et le mélange HCl 0,1N / Ethanol absolu concentré à 20 % (v/v) en éthanol absolu pour les microgranules présentant un taux d'enrobage de 20% sont consignés sur la figure 4.

Le pourcentage de principe actif libéré dans le milieu exempt d'éthanol atteint 70% à 30 minutes puis 93% à 1 heure. Ces microgranules préparés selon l'invention WO2007093642 ne constituent donc pas une forme multiparticulaire à libération prolongée résistants à l'alcool.

### Exemple 4 : Microgranules de Diltiazem HCl à base de neutres de sucre rendus insolubles, à libération prolongée résistants à l'alcool

Selon une variante de l'exemple 1, démontrant que les microgranules de Diltiazem HCl préparés à partir de neutres de sucre ne sont pas résistants à l'alcool, des microgranules de Diltiazem HCl résistants à l'alcool peuvent être obtenus en rendant les neutres de sucre insolubles dans l'eau ou dans une solution alcoolique.

Les neutres de sucres insolubles dans l'eau ou dans une solution alcoolique sont obtenus en procédant au montage d'une couche d'excipients insolubles dans l'eau ou dans une solution alcoolique sur ces neutres. La composition de solution de montage utilisée et les quantités de matières mises en oeuvre sont les suivantes :

| | Quantité (grammes) | Composition centésimale en matière sèche (%) |
|---|---|---|
| Neutres de sucre | 700 | 72 |
| Aquacoat ECD 30 | 533 | |
| *(masse sèche)* | *(160)* | 16 |
| Triéthylcitrate | 40 | 4 |
| Talc | 80 | 8 |
| Eau purifiée | 1213 | N/A |
| Total en masse sèche | 980 | 100 |

Les neutres de sucre rendus insolubles dans l'eau ou dans une solution alcoolique ainsi préparés sont sphériques et voient leur diamètre augmenter d'environ 15% (diamètre compris entre 600 et 800µm) par rapport aux neutres de sucres non enrobés.

Les neutres insolubles sont ensuite mis en oeuvre pour préparer des microgranules de Diltiazem HCl à libération prolongée. Les méthodes de préparation, de dosage et de dissolution de ces microgranules restent identiques à l'exemple 1, à l'exception de leur composition quantitative récapitulée dans le tableau suivant :

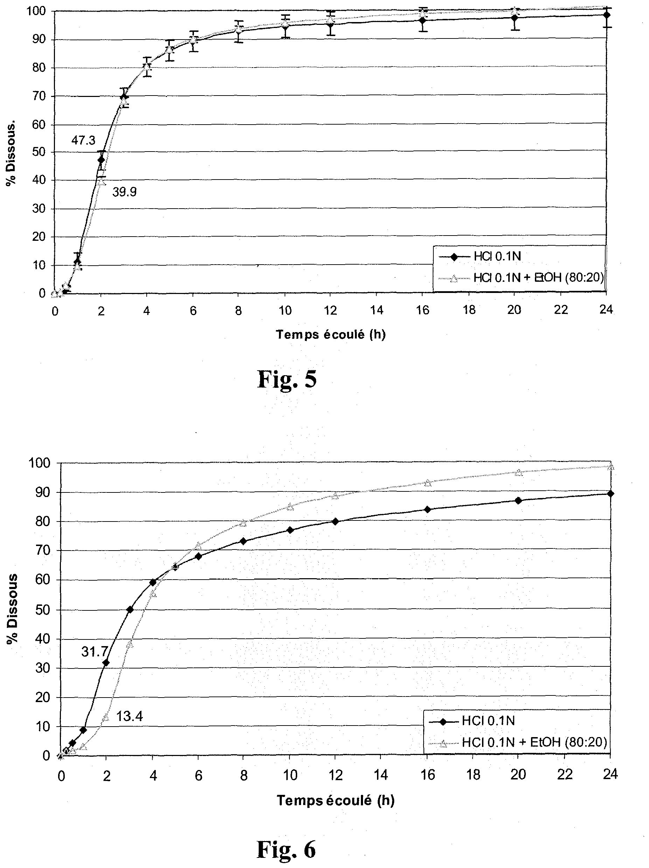

Les profils de dissolution obtenus dans l'HCl 0,1N et le mélange HCl 0,1N / Ethanol absolu concentré à 20% (v/v) en éthanol absolu pour les microgranules préparés avec un taux d'enrobage de 20% sont consignés sur la figure 5.

Dans l'exemple 1, il est montré que les microgranules préparés à partir de neutres de sucre perdent leur propriété de libération prolongée en présence d'éthanol. Sur la figure 5, il apparaît clairement que les microgranules préparés à partir de neutres de sucre rendus insolubles dans l'eau ou dans une solution alcoolique conservent leur propriété de libération prolongée en présence d'alcool. L'écart des pourcentages d'actif libéré au bout de 2 heures dans l'HCl 0,1N par rapport au milieu acido-alcoolique est de -7%, ce qui démontre que ces microgranules LP sont résistants à l'alcool.

### Exemple 5 : Microgranules de Diltiazem HCl à libération prolongée résistants à l'alcool - Effet du plastifiant

| | | Composition centésimale pour obtenir un taux d'enrobage de 20% |
|---|---|---|
| **Neutres rendus insolubles** | Support neutre de sucre | 46,2 |
| | Aquacoat ECD 30 *(masse sèche)* | 10,6 |
| | Triéthylcitrate | 2,6 |
| | Talc | 5,3 |
| **Montage** | PVP K30 | 2,0 |
| | Diltiazem HCl | 16,2 |
| **Enrobage** | Aquacoat ECD 30 *(masse sèche)* | 13,3 |
| | Triéthylcitrate | 3,3 |
| **Lubrification** | Aérosil® R972 | 0,5 |

### Utilisation de la triacétine comme plastifiant :

Selon une variante de l'exemple 1, la préparation de microgranules de Diltiazem HCl résistants à l'alcool peut être réalisée à l'aide de la triacétine (Oleo Chemical) en tant que plastifiant dans la suspension d'enrobage. Deux taux d'enrobage sont présentés : 15 et 20%.

Les méthodes de préparation, de dosage et de dissolution des microgranules restent identiques à l'exemple 1, à l'exception de leurs compositions quantitative et qualitative récapitulées dans le tableau suivant (composition de référence):

| | | Composition centésimale pour obtenir un taux d'enrobage de 15% | Composition centésimale pour obtenir un taux d'enrobage de 20% |
|---|---|---|---|
| **Montage** | Support neutre de cellulose | 67,5 | 64,7 |
| | PVP K30 | 2,1 | 2,0 |
| | Diltiazem HCl | 16,9 | 16,2 |
| **Enrobage** | Aquacoat ECD 30 *(en masse sèche)* | 10,5 | 13,4 |
| | Triacétine | 2,5 | 3,2 |
| **Lubrification** | Aérosil® R972 | 0,5 | 0,5 |

Les profils de dissolution obtenus dans l'HCl 0,1N et le mélange HCl 0,1N / Ethanol absolu concentré à 20 % (v/v) en éthanol absolu pour les microgranules préparés avec un taux d'enrobage de 20 % sont consignés sur la figure 6.

L'écart des pourcentages d'actif libéré de ces microgranules LP au bout de 2 heures dans l'HCl 0,1N par rapport au milieu acido-alcoolique est respectivement de 2 et -18% pour les taux d'enrobage de 15 et 20 %.

### - Utilisation des monoglycérides acétylés comme plastifiant

Selon une variante de l'exemple 1, la préparation de microgranules de Diltiazem HCl résistants à l'alcool peut être réalisée en utilisant comme plastifiants des monoglycérides acétylés, notamment le Myvacet® 9-45 (Kerry). Dans le présent exemple, le taux d'enrobage est de 20%.

Les méthodes de préparation, de dosage et de dissolution des microgranules restent identiques à l'exemple 1, à l'exception de leurs compositions quantitative et qualitative récapitulées dans le tableau suivant (composition de référence):

| | | Composition centésimale pour obtenir un taux d'enrobage de 20% |
|---|---|---|
| **Montage** | Support neutre de cellulose | 64,7 |
| | PVP K30 | 2,0 |
| | Diltiazem HCl | 16,2 |
| **Enrobage** | Aquacoat ECD 30 *(en masse sèche)* | 13,4 |
| | Myvacet® 9-45 | 3,2 |
| **Lubrification** | Aérosil® R972 | 0,5 |

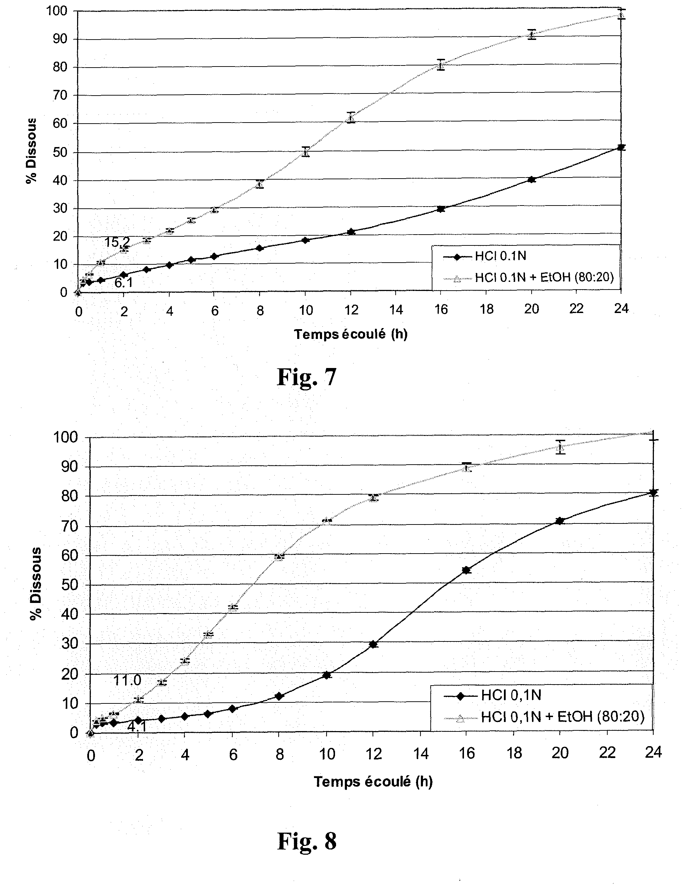

Les profils de dissolution obtenus dans l'HCl 0,1N et le mélange HCl 0,1N / Ethanol absolu concentré à 20 % (v/v) en éthanol absolu pour ces microgranules sont consignés sur la figure 7.

L'écart des pourcentages d'actif libéré au bout de 2 heures dans l'HCl 0,1N par rapport au milieu acido-alcoolique est de 9%, ce qui démontre que ces microgranules LP sont résistants à l'alcool.

### - Utilisation du dibutylsébacate comme plastifiant :

Selon une variante de l'exemple 1, la préparation de microgranules de Diltiazem HCl résistants à l'alcool peut être réalisée en utilisant du dibutylsébacate comme plastifiant dans la suspension d'enrobage. Le taux d'enrobage présenté est de 20%.

Les méthodes de préparation, de dosage et de dissolution des microgranules restent identiques à l'exemple 1, à l'exception de leurs compositions quantitative et qualitative récapitulées dans le tableau suivant (composition de référence):

| | | Composition centésimale pour obtenir un taux d'enrobage de 20% |
|---|---|---|
| **Montage** | Support neutre de cellulose | 64,7 |
| | PVP K30 | 2,0 |
| | Diltiazem HCl | 16,2 |
| **Enrobage** | Aquacoat ECD 30 *(en masse sèche)* | 13,4 |
| | Dibutylsébacate | 3,2 |
| **Lubrification** | Aérosil® R972 | 0,5 |

Les profils de dissolution obtenus dans l'HCl 0,1N et le mélange HCl 0,1N / Ethanol absolu concentré à 20 % (v/v) en éthanol absolu pour ces microgranules sont consignés sur la figure 8.

L'écart des pourcentages d'actif libéré au bout de 2 heures dans l'HCl 0,1N par rapport au milieu acido-alcoolique est de 7%, ce qui démontre que ces microgranules LP sont résistants à l'alcool.

### Exemple 6 : Microgranules de Carvédilol Phosphate à libération prolongée résistants à l'alcool (exemple de référence)

### a) Préparation des microgranules de Carvedilol Phosphate à libération prolongée

Le principe actif utilisé est le Carvedilol phosphate hemihydrate (Zambon), de formule C₂₄H₂₆N₂O₄.H₃PO₄. ½H₂O. Les noyaux neutres utilisés sont des sphères de sucre (Suglets® 30 NPPHARM) et des sphères de cellulose (Ethisphères® 600 NPPHARM). La taille de ces supports est de l'ordre de 500 à 700µm. L'agent liant utilisé est la polyvinylpyrrolidone (PVP K 30, BASF) et plusieurs tensioactifs sont utilisés pour stabiliser la suspension de montage : Simethicone (Emulsion 30%, Dow Corning) et Polysorbate 80 (Seppic).

La composition de suspension de montage et les quantités de matières mises en oeuvre pour l'étape de montage sont les suivantes :

| | Quantité (grammes) | | Composition centésimale en matière sèche (%) | |
|---|---|---|---|---|
| | Neutres de sucre | Neutres de cellulose | Neutres de sucre | Neutres de cellulose |
| Noyaux neutres | 1000 | 1000 | 68,2 | 62,8 |
| PVP K 30 | 150 | 240 | 10,2 | 15,1 |
| Polysorbate 80 | 15 | 32 | 1,0 | 2,0 |
| Simethicone (émulsion 30%) | 1 | 1 | | |
| *(masse sèche)* | *(0,3)* | *(0,3)* | 0,02 | 0,02 |
| Carvedilol Phosphate | 300 | 320 | 20,5 | 20,1 |
| Eau purifiée | 1832 | 1943 | N/A | N/A |
| Total en masse sèche | 1465,3 | 1592,3 | 100 | 100 |

| | | | | |
|---|---|---|---|---|
| N/A : non applicable | | | | |

Les noyaux neutres sont introduits dans un lit fluidisé (GPCG1, Glatt) équipé d'un Würster. La suspension de montage est pulvérisée en bottom spray avec une buse de diamètre 1,2mm.

Un tamisage avec des grilles d'ouverture de mailles 500 et 900µm est réalisé après le montage afin d'éliminer respectivement les fines et les doubles.

La suspension d'enrobage est réalisée à partir d'une préparation commerciale d'Aquacoat® ECD 30 (FMC) à laquelle est ajouté un plastifiant, le triéthylcitrate (TEC). La suspension est ensuite diluée afin d'obtenir une dispersion aqueuse à 15% de masse sèche.

La composition de la suspension d'enrobage finale est la suivante :

| | Composition centésimale(%) | Composition centésimale en matière sèche (%) |
|---|---|---|
| Aquacoat® ECD 30 | 40,3 | 15 |
| *(masse sèche)* | *(12,09)* | |
| Triéthylcitrate | 2,9 | |
| Eau purifiée | 56,8 | N/A |

La suspension d'enrobage est pulvérisée sur les microgranules montés avec le carvedilol phosphate.

Les quantités de matières mises en oeuvre pour l'étape d'enrobage sont les suivantes :

| | Quantité (grammes) pour obtenir un taux d'enrobage de 5 % |
|---|---|
| Noyaux montés avec le Carvedilol Phosphate | 750,0 |
| Suspension d'enrobage à 15% matière sèche | 250,0 |
| *Masse sèche* | *37,5* |
| Total en masse sèche | 787,5 |

Le lit fluidisé (GPCG1, Glatt) est équipé d'un Würster. La pulvérisation s'effectue en bottom spray avec une buse de diamètre 1,2mm. Un tamisage avec des grilles d'ouverture de mailles 500µm et 1000µm est réalisé après l'enrobage afin d'éliminer respectivement les fines et les doubles.

Une maturation du film d'enrobage est réalisée en étuve (FIRLABO) 24h à 60°C et avec 75% d'humidité relative (HR).

Les compositions qualitative et quantitative des microgranules de Carvédilol sont récapitulées dans le tableau suivant.

| | | Composition centésimale (%) pour obtenir un taux d'enrobage de 5% | |
|---|---|---|---|
| | | Neutres de sucre | Neutres de cellulose |
| **Montage** | Noyaux neutres | 64,7 | 59,5 |
| | PVP K30 | 9,7 | 14,3 |
| | Polysorbate 80 | 1,0 | 1,9 |
| | Simethicone *(masse sèche)* | 0,02 | 0,02 |
| | Carvedilol Phosphate | 19,4 | 19,0 |
| **Enrobage** | Aquacoat ECD 30 *(masse sèche)* | 3,8 | 3,8 |
| | Triéthylcitrate | 0,9 | 0,9 |
| **Lubrification** | Aérosil® R972 | 0,5 | 0,5 |

### b) Dosage et dissolution des microgranules

Les essais de libération *in vitro* du principe actif sont réalisés dans un appareil de dissolution à pales tournantes (Pharmacopée Européenne, Sotax AT7, logiciel IDIS). L'analyse se fait avec un spectrophotomètre UV / visible (Kontron Instruments spectrophotometer, UVIKON 922), à une longueur d'onde de 241 nm.

Les échantillons sont soumis à une agitation constante dans des vases contenant chacun 900mL de milieu de dissolution, et la température est maintenue constante à 37°C (+/-0,5°C), Les milieux de dissolution utilisés sont composés soit d'HCl 0,1N, soit d'un mélange HCl 0,1N / Ethanol absolu avec une concentration en éthanol absolu égale à 20 % (v/v). La vitesse de rotation des pales est fixée à 100 tours/min.

Des prélèvements sont réalisés en continu sur 24h dans chacun des 6 vases de l'appareil. Pour chaque lot, le test est réalisé sur 3 vases, avec 3 prises d'essai de microgranules équivalentes chacune à 80 mg de PA.

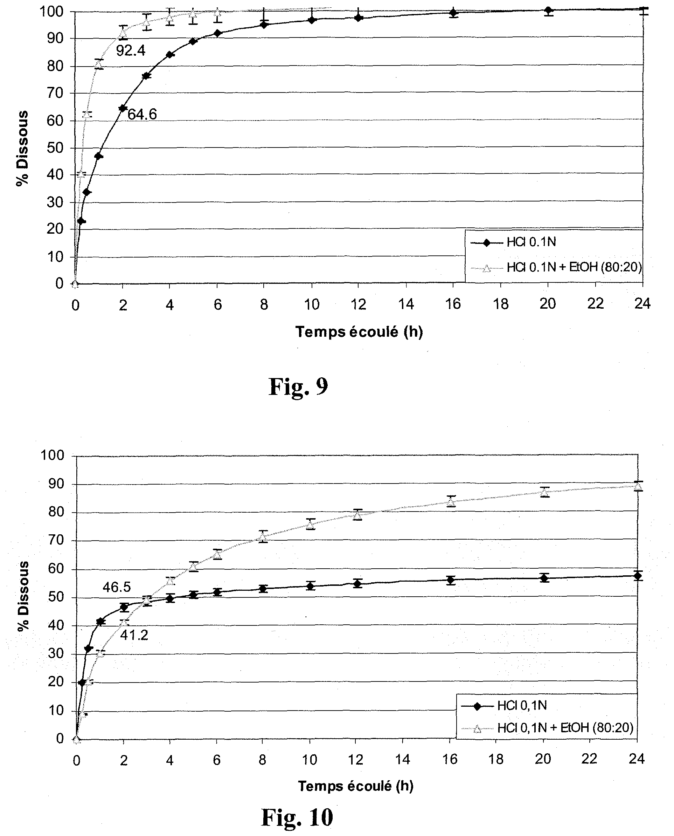

Les profils de dissolution obtenus dans l'HCl 0,1N et le mélange HCl 0,1N / Ethanol absolu concentré à 20 % (v/v) en éthanol absolu pour ces microgranules sont consignés sur les figures 9 et 10.

La figure 9 montre que la vitesse de libération du Carvédilol phosphate monté sur neutres de sucre est très accélérée en présence d'éthanol par rapport au milieu composé d'HCl 0,1N seul. L'écart des pourcentages de Carvédilol Phosphate libéré de ces microgranules LP au bout de 2 heures est de 28%. Ces microgranules LP ne sont donc pas résistants à l'alcool.

A l'inverse, les systèmes à base de neutres de cellulose conservent leur propriété de libération prolongée du Carvédilol phosphate en présence d'éthanol (Figure 10). L'écart des pourcentages de Carvédilol Phosphate libéré de ces microgranules LP au bout de 2 heures dans l'HCl 0,1N par rapport au milieu acido-alcoolique est de -5%, ce qui démontre la résistance de ce système à l'alcool.

### Exemple 7 : Microgranules de Carvédilol Phosphate à libération prolongée résistants à l'alcool préparés à partir de supports de sucre rendus insolubles

Selon une variante de l'exemple 8, démontrant que les microgranules de Carvédilol Phosphate préparés à partir de neutre de sucre ne sont pas résistants à l'alcool, des microganules de Carvédilol Phosphate résistants à l'alcool peuvent être obtenus en rendant les neutres de sucre insolubles dans l'eau ou dans une solution alcoolique.

Les neutres de sucres insolubles dans l'eau ou dans une solution alcoolique sont obtenus en procédant au montage d'une couche d'excipients insolubles dans l'eau ou dans une solution alcoolique sur ces neutres. La composition de solution de montage utilisée et les quantités de matières mises en oeuvre sont les suivantes :

| | Quantité (grammes) | Composition centésimale en matière sèche (%) |
|---|---|---|
| Neutres de sucre | 700 | 72 |
| Aquacoat ECD 30 | 533 | |
| *(masse sèche)* | *(160)* | 16 |
| Triéthylcitrate | 40 | 4 |
| Talc | 80 | 8 |
| Eau purifiée | 1213 | N/A |
| Total en masse sèche | 980 | 100 |

Les neutres de sucre rendus insolubles dans l'eau ou dans une solution alcoolique ainsi préparés sont sphériques et voient leur diamètre augmenter d'environ 15% (diamètre compris entre 600 et 800µm) par rapport aux neutres de sucres non enrobés.

Les neutres insolubles sont mis en oeuvre pour préparer des microgranules de Carvédilol phosphate à libération prolongée. Les méthodes de préparation, de dosage et de dissolution de ces microgranules restent identiques à l'exemple 8, à l'exception de leur composition quantitative récapitulée dans le tableau suivant :

| | | Composition centésimale pour obtenir un taux d'enrobage de 5% |
|---|---|---|
| **Enrobage des neutres** | Support neutre de sucre | 40,0 |
| | Aquacoat ECD 30 *(masse sèche)* | 9,2 |
| | Triéthylcitrate | 2,3 |
| | Talc | 4,6 |
| **Montage** | PVP K30 | 12,5 |
| | Polysorbate 80 | 1,3 |
| | Simethicone *(masse sèche)* | 0,02 |
| | Carvedilol Phosphate | 24.9 |
| **Enrobage** | Aquacoat ECD 30 *(masse sèche)* | 3,8 |
| | Triéthylcitrate | 0,9 |
| **Lubrification** | Aérosil® R972 | 0,5 |

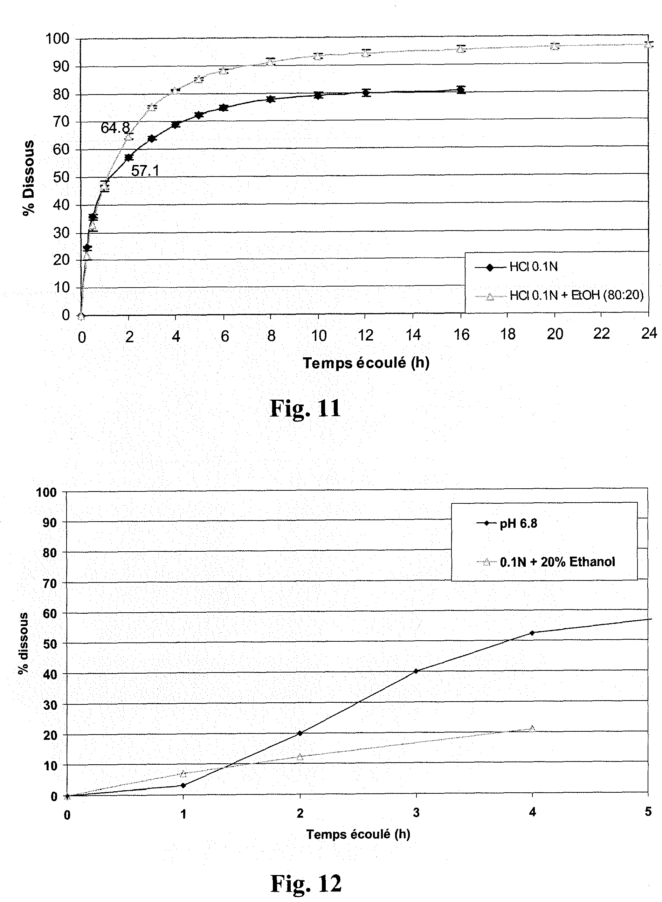

Les profils de dissolution obtenus dans l'HCl 0,1N et le mélange HCl 0,1N / Ethanol absolu concentré à 20 % (v/v) en éthanol absolu pour les microgranules préparés avec un taux d'enrobage de 20 % sont consignés sur la figure 11.

Dans l'exemple 6, il est montré que les microgranules préparés à partir de neutres de sucre perdent leur propriété de libération prolongée en présence d'éthanol. Sur la figure 11, il apparaît clairement que les microgranules préparés à partir de neutres de sucre rendus insolubles dans l'eau ou dans une solution alcoolique conservent leur propriété de libération prolongée en présence d'alcool. L'écart des pourcentages d'actif libéré au bout de 2 heures dans l'HCl 0,1N par rapport au milieu acido-alcoolique est de 8%, ce qui démontre que ces microgranules LP sont résistants à l'alcool.

### Exemple 8 : Microgranules de Toltérodine à libération prolongée résistants à l'alcool (exemple de référence)

### a) Préparation des microgranules de Toltérodine tartrate à libération prolongée

Les noyaux neutres utilisés sont des sphères de cellulose (Celpheres 507, Asahi Kasei), dont la taille est l'ordre de 500 à 710µm. L'agent liant utilisé est l'hypromellose 606 (Pharmacoat 606, Shin-Etsu).

| | Composition | |
|---|---|---|
| | Composition massique (g) | Composition centésimale (%) |
| Celphere 507 | 744,0 | 96,62 |
| Tolterodine Tartrate | 21,0 | 2,73 |
| Pharmacoat 606 | 5,0 | 0,65 |
| *Eau purifiée* | *1400,0* | |
| Total masse sèche | 770,0 | 100,00 |

Les noyaux neutres sont introduits dans un lit fluidisé (GPCG1, Glatt) équipé d'un Würster. La suspension de montage est pulvérisée en bottom spray avec une buse de diamètre 1,2mm.

Un tamisage avec des grilles d'ouverture de mailles 500 et 900µm est réalisé après le montage afin d'éliminer respectivement les fines et les doubles.

La suspension d'enrobage est réalisée à partir d'une préparation commerciale de Surelease (Colorcon). Il s'agit d'une dispersion aqueuse à 25% d'éthylcellulose prête à l'emploi, pré-plastifiée par des triglycérides à chaînes moyennes, comprenant des stabilisants et co-stabilisants (acide oléique, solution aqueuse d'hydroxyde d'ammonium) et éventuellement un anti-adhérent (silice colloïdale). La suspension d'enrobage est pulvérisée sur les microgranules montés avec la tolterodine. Les quantités de matières mises en oeuvre pour l'étape d'enrobage sont les suivantes :

| | Composition | |
|---|---|---|
| | Composition massique (g) | Composition centésimale (%) |
| Neutres montés en toltérodine | 390,00 | 73,00 |
| Surelease | 480,84 | |
| *(masse sèche)* | *(120,21* | 22,50 |
| Pharmacoat 606 | 24,04 | 4,50 |
| *Eau purifiée* | *480,84* | *N*/*A* |
| Total masse sèche | 534,25 | 100,00 |

Le lit fluidisé (GPCG1, Glatt) est équipé d'un Würster. La pulvérisation s'effectue en bottom spray avec une buse de diamètre 1,2mm. Un tamisage avec des grilles d'ouverture de mailles 500µm et 1000µm est réalisé après l'enrobage afin d'éliminer respectivement les fines et les doubles.

Une maturation du film d'enrobage est réalisée en étuve 4h à 55°.

Les compositions qualitative et quantitative des microgranules de Toltérodine sont récapitulées dans le tableau suivant.

| | Composition relative (%) |
|---|---|
| Celphere 507 | 70,53 |
| Tolterodine Tartrate | 2,00 |
| Pharmacoat 606 | 4,97 |
| Surelease (masse sèche) | 22,50 |
| Total | 100,00 |

### b) Dosage et dissolution des microgranules

Les essais de libération *in vitro* du principe actif sont réalisés dans un appareil de dissolution équipé de paniers (Pharmacopée Européenne).

Les échantillons sont soumis à une agitation constante dans des vases contenant chacun 900mL de milieu de dissolution, et la température est maintenue constante à 37°C (+/-0,5°C), Les milieux de dissolution utilisés sont composés soit d'un milieu pH 6,8, soit d'un mélange HCl 0,1N / Ethanol absolu avec une concentration en éthanol absolu égale à 20% (v/v). La vitesse de rotation des paniers est fixée à 100 tours/min.

Des prélèvements sont réalisés sur les quatre premières heures de dissolution dans chacun des 6 vases de l'appareil. Pour chaque lot, le test est réalisé sur 3 vases, avec 3 prises d'essai de microgranules équivalentes chacune à 4 mg de PA.

Les profils de dissolution obtenus dans le milieu pH 6,8 et le mélange HCl 0,1N / Ethanol absolu concentré à 20 % (v/v) en éthanol absolu pour ces microgranules sont consignés sur la figure 12.

L'écart des pourcentages de Toltérodine libérée au bout de 2 heures dans un milieu aqueux et dans le milieu hydro-alcoolique est de -7,7%, indiquant que ce système est résistant à la présence d'alcool dans le milieu de dissolution.

## Revendications

1. Forme pharmaceutique orale à base de microgranules à libération prolongée d'au moins un principe actif, comprenant
- un support neutre rendu insoluble dans l'eau ou dans une solution alcoolique, comportant
- au moins une première couche de montage comprenant au moins un principe actif et éventuellement un agent liant pharmaceutiquement acceptable ; l'ensemble comportant
- au moins un enrobage à base d'au moins un polymère hydrophobe,
pour son utilisation dans une méthode thérapeutique pour éviter ou limiter une décharge immédiate du principe actif induite par la consommation d'alcool, ladite forme pharmaceutique étant **caractérisée en ce que** le support neutre rendu insoluble est obtenu en recouvrant un support neutre d'un ou plusieurs excipients de nature hydrophobe et choisis parmi les dérivés de la cellulose, et **en ce que** ladite forme pharmaceutique comporte au moins un plastifiant dans l'enrobage.

2. Forme pharmaceutique pour son utilisation selon la revendication 1, **caractérisée en ce que** le pourcentage d'actif libéré après 2h dans un milieu acido-alcoolique HCl 0,1N contenant de l'alcool et de préférence une quantité d'éthanol comprise entre 4 et 30%, n'est pas supérieur de plus de 15 points comparé à celui libéré dans un milieu aqueux acide HCl 0,1N.

3. Forme pharmaceutique pour son utilisation selon l'une quelconque des revendications 1 à 2, **caractérisée en ce que** l'agent liant pharmaceutiquement acceptable est choisi dans le groupe constitué par les dérivés de la cellulose tels que l'HPMC, les dérivés de la polyvinylpyrrolidone, et également les dérivés du polyéthylene glycol, et leurs mélanges.

4. Forme pharmaceutique pour son utilisation selon l'une quelconque des revendications 1 à 3, **caractérisée en ce que** ladite forme pharmaceutique comporte au moins un agent tensio-actif dans la couche de montage.

5. Forme pharmaceutique pour son utilisation selon l'une quelconque des revendications 1 à 4, **caractérisée en ce que** le polymère hydrophobe de l'enrobage est choisi dans le groupe constitué par : les dérivés non hydrosolubles de la cellulose, les dérivés de (co)polymères (méth)acryliques, les dérivés de polyvinylacétate et leurs mélanges.

6. Forme pharmaceutique pour son utilisation selon l'une quelconque des revendications 1 à 5, **caractérisée en ce que** le taux d'enrobage est compris entre 0,1% à 50% m/m, de préférence, de 2% à 30% m/m, et, plus préférentiellement encore, de 5% à 30%.

7. Forme pharmaceutique pour son utilisation selon l'une quelconque des revendications 1 à 6, **caractérisée en ce que** le principe actif est choisi parmi les hormones ou leurs dérivés, les principes actifs agissant sur le système nerveux central, les principes actifs agissant sur le système cardiovasculaire, les antibiotiques, les anti-viraux et les analgésiques.

## Patentansprüche

1. Orale pharmazeutische Form auf der Basis von Mikrogranulaten mit verzögerter Freisetzung von zumindest einem Wirkstoff, die Folgendes umfasst
- einen neutralen Träger, der in Wasser oder in einer alkoholischen Lösung unlöslich gemacht wird, der
- zumindest eine erste Befestigungsschicht aufweist, die mindestens ein Wirkstoff und eventuell ein pharmazeutisch verträgliches Bindemittel enthält; wobei die Anordnung
- zumindest eine Beschichtung auf Basis von mindestens einem hydrophoben Polymer enthält,
zur Verwendung in einem therapeutischen Verfahren, um eine durch Alkoholkonsum induzierte sofortige Freisetzung des Wirkstoffs zu vermeiden oder zu begrenzen, wobei die pharmazeutische Form **dadurch gekennzeichnet ist, dass** der unlöslich gemachte neutrale Träger mit einem neutralen Träger mit einem oder mehreren Hilfsstoffen hydrophober Natur beschichtet wird, die unter Cellulosederivaten ausgewählt werden, und dass die pharmazeutische Form in der Beschichtung zumindest einen Weichmacher enthält.

2. Pharmazeutische Form zur Verwendung nach Anspruch 1, **dadurch gekennzeichnet, dass** der Prozentsatz des Wirkstoffs, der nach 2 Stunden in einem säurealkoholischen HCl 0,1 N Milieu freigesetzt wurde, das Alkohol und vorzugsweise einer Menge Ethanol zwischen 4 und 30 % enthält, nicht mehr als 15 Punkte höher ist als der in einem wässrigen sauren HCl 0,1 N Milieu freigesetzte Prozentsatz.

3. Pharmazeutische Form zur Verwendung nach einem der Ansprüche 1 bis 2, **dadurch gekennzeichnet, dass** das pharmazeutisch verträgliche Bindemittel aus der Gruppe bestehend aus Cellulosederivaten wie HPMC, Polyvinylpyrrolidonderivaten und auch Polyethylenglykolderivaten und Mischungen davon ausgewählt ist.

4. Pharmazeutische Form zur Verwendung nach einem der Ansprüche 1 bis 3, **dadurch gekennzeichnet, dass** die pharmazeutische Form mindestens ein Tensid in der Befestigungsschicht umfasst.

5. Pharmazeutische Form zur Verwendung nach einem der Ansprüche 1 bis 4, **dadurch gekennzeichnet, dass** das hydrophobe Polymer der Beschichtung aus der Gruppe bestehend aus: wasserunlösliche Cellulosederivate, (Co)polymer(meth)acryl-Derivate, Polyvinylacetat-Derivate und Mischungen davon ausgewählt ist.

6. Pharmazeutische Form zur Verwendung nach einem der Ansprüche 1 bis 5, **dadurch gekennzeichnet, dass** die Beschichtungsrate zwischen 0,1 % und 50 % m/m, vorzugsweise zwischen 2 % und 30 % m/m, und noch bevorzugter zwischen 5 % und 30 % liegt.

7. Pharmazeutische Form zur Verwendung nach einem der Ansprüche 1 bis 6, **dadurch gekennzeichnet, dass** der Wirkstoff aus Hormonen oder deren Derivaten, Wirkstoffen, die auf das zentrale Nervensystem wirken, Wirkstoffen, die auf das Herz-Kreislauf-System wirken, Antibiotika, antiviralen Stoffen und Analgetika ausgewählt ist.

## Claims

1. An oral pharmaceutical form containing microgranules for the sustained release of at least one active principle, comprising
- a neutral carrier rendered insoluble in water or in an alcohol solution, comprising
- at least one first mounting layer comprising at least one active principle and optionally a pharmaceutically acceptable binder; the whole comprising
- at least one coating based on at least one hydrophobic polymer,
for use in a therapeutic method to prevent or limit an immediate release of the active principle induced by the consumption of alcohol, said pharmaceutical form being **characterized in that** the neutral carrier rendered insoluble is obtained by covering a neutral carrier with one or more hydrophobic excipients and selected from the cellulose derivatives, and **in that** the pharmaceutical form comprises at least one plasticizer in the coating.

2. The pharmaceutical form for use according to claim 1, **characterized in that** the percentage of active principle released after 2 h in an acid-alcohol medium of 0.1 N HCl containing alcohol and preferably a quantity of ethanol between 4% and 30%, is not more than 15 points greater than that released in an acid aqueous medium of 0.1 N HCl.

3. The pharmaceutical form for use according to any of claims 1 to 2, **characterized in that** the pharmaceutically acceptable binder is chosen from the group constituted by cellulose derivatives such as HPMC, polyvinylpyrrolidone derivatives, and also polyethylene glycol derivatives, and mixtures thereof.

4. The pharmaceutical form for use according to any of claims 1 to 3, **characterized in that** said form comprises at least one surfactant in the mounting layer.

5. The pharmaceutical form for use according to any of claims 1 to 4, **characterized in that** the hydrophobic polymer of the coating is selected from the group constituted by: non-water-soluble cellulose derivatives, (meth)acrylic (co)polymer derivatives, polyvinyl acetate derivatives and mixtures thereof.

6. The pharmaceutical form for use according to any of claims 1 to 5, **characterized in that** the coating ratio is from 0.1% to 50% w/w, preferably from 2% to 30% w/w, and more preferentially still from 5% to 30% w/w.

7. The pharmaceutical form for use according to any of claims 1 to 6, **characterized in that** the active principle is selected from hormones or derivatives thereof, active principles that act on the central nervous system, active principles that act on the cardiovascular system, antibiotics, antivirals and analgesics.
